# EUROPEAN PATENT APPLICATION

(11) **EP 4 660 316 A1**
(43) Date of publication of application: **10.12.2025**
(21) Application number: 24750235.4
(22) Date of filing: 30.01.2024
(51) Int. Cl.: C12Q 1/02

(54) **DRUG EVALUATION METHOD, REAGENT AND KIT**

(30) Priority: 31.01.2023 JP 2023012406; 29.05.2023 JP 2023087432
(71) Applicant: FUJIFILM Corporation, Tokyo 106-8620 (JP)
(72) Inventor: WATANABE Eriko, Ashigarakami-gun, Kanagawa 258-8577 (JP); MIYOSHI Hayato, Ashigarakami-gun, Kanagawa 258-8577 (JP); KATO Hiroshi, Ashigarakami-gun, Kanagawa 258-8577 (JP); MOCHIZUKI Nobuyuki, Ashigarakami-gun, Kanagawa 258-8577 (JP); HARA Reiko, Ashigarakami-gun, Kanagawa 258-8577 (JP)
(74) Representative: Hoffmann Eitle
(86) International application number: PCT/JP2024/002722
(87) International publication number: WO 2024/162286

(57) **Abstract**

An object of the present invention is to provide a drug evaluation method using myocardial cells, which can stably evaluate an effect of a drug such as a muscarinic receptor agonist, an adrenergic receptor inhibitor or a muscarinic receptor inhibitor on a heart rate; and a reagent and a kit for carrying out the drug evaluation method. According to the present invention, there is provided a drug evaluation method including: treating myocardial cells with a culture medium containing a physiologically active substance of a sympathetic nervous system and/or a physiologically active substance of a parasympathetic nervous system; bringing a drug into contact with the myocardial cells treated as above; and evaluating an effect of the drug on a heart rate of the myocardial cells.

## Description

### Technical Field

The present disclosure relates to a drug evaluation method, including evaluating an effect of a drug on a heart rate of myocardial cells, using the myocardial cells. The present disclosure further relates to a reagent and a kit for carrying out the above-mentioned drug evaluation method.

### Background Art

One of the methods for diagnosing a heart disease is to measure a beating rate of myocardial cells. In addition, for the purpose of screening for a drug, the drug efficacy evaluation or toxicity evaluation of a drug is carried out using myocardial cells induced to be differentiated from pluripotent stem cells or primary myocardial cells established from a living body.

For example, Patent Document 1 describes that the evaluation of an increase and a decrease in heart rate by an adrenergic receptor agonist or a muscarinic receptor agonist can be carried out using primate pluripotent stem cell-derived myocardial cells. In Non-Patent Document 1, a decrease in heart rate by a muscarinic receptor agonist and an increase in heart rate by a muscarinic receptor inhibitor are described using mouse-derived primary myocardial cells. Furthermore, in Non-Patent Document 2, it is described that a drug is evaluated using iPS cell-derived myocardial cells, and a decrease in heart rate by a muscarinic receptor agonist is described.

Patent Document 2 describes the induction of myocardial cell differentiation by co-culturing human embryonic stem (hES) cells with cells that secrete a myocardial cell differentiation-inducing factor or an extracellular culture medium obtained therefrom under differentiation-inducing conditions, and describes the testing of the effects of phenylephrine, isoprenaline, and carbachol on cultured dissociated hES myocardial cells.

Patent Document 3 describes a method for producing a contractile cell construct, which includes a step of seeding pre-selected cells containing signal-emitting particles onto a silicone mold and a step of culturing the pre-selected cells to produce a contractile cell construct.

### Prior Art Documents

### Patent Documents

Patent Document 1: JP5917429B
Patent Document 2: JP2006-523091A
Patent Document 3: US2018/0044640A

### Non-Patent Documents

Non-Patent Document 1: Kohashi et al., Biological Rhythm Research, 34 (4), 367-381, 2004
Non-Patent Document 2: Lu et al., Frontiers in Physiology, 13, Article 838435, 2022

### SUMMARY OF THE INVENTION

### Object to be solved by the invention

In the studies of the present inventors, it has been revealed that, in the related art evaluation method using myocardial cells, there is a case where it is difficult to evaluate a muscarinic receptor agonist. In addition, it has been revealed that, in the related art evaluation method using myocardial cells, it is not possible to evaluate adrenergic receptor and muscarinic receptor inhibitors.

An object of the present disclosure is to provide a drug evaluation method using myocardial cells, which is capable of evaluating an effect of a drug, such as a muscarinic receptor agonist, an adrenergic receptor inhibitor, or a muscarinic receptor inhibitor, on a heart rate with high throughput. Another object of the present disclosure is to provide a reagent and a kit for carrying out the above-mentioned drug evaluation method.

### Means for solving the object

As a result of intensive studies to achieve the above-mentioned objects, the present inventors have found that, in a method for evaluating an effect of a drug on a heart rate of myocardial cells by bringing the myocardial cells into contact with the drug, the drug can be evaluated with high throughput by treating the myocardial cells with a culture medium containing a physiologically active substance of the sympathetic nervous system and/or a physiologically active substance of the parasympathetic nervous system before the myocardial cells are brought into contact with the drug. The present disclosure has been completed based on the above findings.

That is, according to the present disclosure, the following inventions are provided.
<1> A drug evaluation method, comprising: treating myocardial cells with a culture medium containing a physiologically active substance of a sympathetic nervous system and/or a physiologically active substance of a parasympathetic nervous system; bringing a drug into contact with the myocardial cells treated as above; and evaluating an effect of the drug on a heart rate of the myocardial cells.
<2> The method according to <1>, in which the culture medium is serum-free.
<3> The method according to <1> or <2>, in which the physiologically active substance of the sympathetic nervous system includes an adrenergic receptor agonist.
<4> The method according to <1> or <2>, in which the physiologically active substance of the parasympathetic nervous system includes a muscarinic receptor agonist.
<5> The method according to <1> or <2>, further comprising: evaluating an inhibitory effect of the drug on an adrenergic receptor.
<6> The method according to <1> or <2>, further comprising: evaluating an inhibitory effect of the drug on a muscarinic receptor.
<7> The method according to <1> or <2>, in which the myocardial cells are human iPS cell-derived myocardial cells.
<8> The method according to <1> or <2>, in which the physiologically active substance of the parasympathetic nervous system is carbachol, and the physiologically active substance of the sympathetic nervous system is isoproterenol.
<9> A reagent for carrying out the method according to <1> or <2>, the reagent comprising an adrenergic receptor agonist and/or a muscarinic receptor agonist.
<10> A kit comprising the reagent according to <9> and a culture medium.
<11> The kit according to <10>, further comprising myocardial cells.

### Effect of the invention

According to the present disclosure, it is possible to evaluate effects of adrenergic receptor and muscarinic receptor agonists, and adrenergic receptor and muscarinic receptor inhibitors on a heart rate with high throughput, using myocardial cells.

### BRIEF DESCRIPTION OF THE DRAWINGS

[Fig. 1] Fig. 1 shows the measurement results of a beating time (seconds).
[Fig. 2] Fig. 2 shows the results of evaluating an effect on a heart rate in a case where a muscarinic receptor agonist (carbachol) is added to a serum-free culture medium.
[Fig. 3] Fig. 3 shows the results of evaluating a decrease in heart rate by a muscarinic receptor agonist (carbachol) using a serum-free culture medium to which an adrenergic receptor agonist (isoproterenol) is added.
[Fig. 4] Fig. 4 shows the results of evaluating a decrease in heart rate by a muscarinic receptor agonist (cevimeline) using a serum-free culture medium to which an adrenergic receptor agonist (isoproterenol) is added.
[Fig. 5] Fig. 5 shows the results of evaluating a decrease in heart rate by a muscarinic receptor agonist (pilocarpine) using a serum-free culture medium to which an adrenergic receptor agonist (isoproterenol) is added.
[Fig. 6] Fig. 6 shows the results of evaluating a decrease in heart rate by carbachol using a maintenance culture medium.
[Fig. 7] Fig. 7 shows the results of evaluating an increase in heart rate by a muscarinic receptor inhibitor (atropine) using a serum-free culture medium to which an adrenergic receptor agonist (isoproterenol) and a muscarinic receptor agonist (carbachol) are added.
[Fig. 8] Fig. 8 shows the results of evaluating an increase in heart rate by a muscarinic receptor inhibitor (tiotropium) using a serum-free culture medium to which an adrenergic receptor agonist (isoproterenol) and a muscarinic receptor agonist (carbachol) are added.
[Fig. 9] Fig. 9 shows the results of evaluating an increase in heart rate by a muscarinic receptor inhibitor (glycopyrronium) using a serum-free culture medium to which an adrenergic receptor agonist (isoproterenol) and a muscarinic receptor agonist (carbachol) are added.
[Fig. 10] Fig. 10 shows the results of evaluating a decrease in heart rate by an adrenergic receptor inhibitor (propranolol) using a serum-free culture medium to which an adrenergic receptor agonist (isoproterenol) and a muscarinic receptor agonist (carbachol) are added.
[Fig. 11] Fig. 11 shows the results of evaluating a decrease in heart rate by an adrenergic receptor inhibitor (atenolol) using a serum-free culture medium to which an adrenergic receptor agonist (isoproterenol) and a muscarinic receptor agonist (carbachol) are added.
[Fig. 12] Fig. 12 shows the results of evaluating an effect in a case where a drug (aspirin) that has no effect on the heart rate is added using a serum-free culture medium to which an adrenergic receptor agonist (isoproterenol) and a muscarinic receptor agonist (carbachol) are added.
[Fig. 13] Fig. 13 shows a method and results of evaluating an increase in heart rate by a muscarinic receptor inhibitor (atropine) using a maintenance culture medium.
[Fig. 14] Fig. 14 shows a method and results of evaluating an increase in heart rate by a muscarinic receptor inhibitor (atropine) using a serum-free culture medium.

### EMBODIMENTS FOR CARRYING OUT THE INVENTION

Hereinafter, the contents of the present disclosure will be described in detail. In the present specification, the numerical range indicated by using "to" means a range that includes numerical values described before and after "to" as a minimum value and a maximum value, respectively. µM is synonymous with µmol/L.

The drug evaluation method according to the present disclosure includes: treating myocardial cells with a culture medium containing a physiologically active substance of the sympathetic nervous system and/or a physiologically active substance of the parasympathetic nervous system; bringing a drug into contact with the myocardial cells treated as above; and evaluating an effect of the drug on a heart rate of the myocardial cells.

The myocardial cells in the present disclosure may be either myocardial cells induced from pluripotent stem cells or myocardial cells established from a tissue (preferably, primary myocardial cells), and are preferably myocardial cells induced from pluripotent stem cells.

The pluripotent stem cells refer to cells having pluripotency, which enables differentiation into all cells that constitute a living body, and a self-replication ability, which can maintain the pluripotency even after cell division. Examples of the pluripotent stem cells include induced pluripotent stem cells (iPS cells), embryonic stem cells (ES cells), and embryoid germ cells (EG cells). Preferably, the pluripotent stem cells are induced pluripotent stem cells (iPS cells) or embryonic stem cells (ES cells). That is, the myocardial cells in the present disclosure are preferably induced pluripotent stem cell (iPS cell)-derived myocardial cells or embryonic stem cell (ES cell)-derived myocardial cells, and more preferably iPS cell-derived myocardial cells.

The biological species of the myocardial cells is not particularly limited, and is preferably a mammal, more preferably a rodent (mouse, rat, hamster, or the like) or a primate (monkey, human, or the like), still more preferably a primate, and particularly preferably a human.

It is particularly preferable that the myocardial cells are human iPS cell-derived myocardial cells.

Methods for inducing myocardial cells from pluripotent stem cells are known, and include, for example, the methods described in WO2013/111875A, WO2015/182765A, WO2020/027278A, and "Minami I, et al., Cell Rep. 2012 Nov 29; 2(5): 1448-60". In addition, the myocardial cells induced from pluripotent stem cells may be cells commercially available as ES cell- or iPS cell-derived myocardial cells (for example, iCell (registered trademark) myocardial cells (manufactured by FUJIFILM Cellular Dynamics, Inc.) or iCell (registered trademark) myocardial cells 2.0 (manufactured by FUJIFILM Cellular Dynamics, Inc.)).

The myocardial cells used in the present disclosure are cultured cells and preferably plane cultured cells, which are preferably myocardial cells in the form of a two-dimensional sheet. That is, the myocardial cells used in the present disclosure are preferably not organoids, which are three-dimensional tissues. By using plane cultured cells instead of organoids, the evaluation of a drug can be easily carried out.

The myocardial cells used in the present disclosure preferably include ventricular cells, atrial cells, and nodal cells. It should be noted that cells called pacemaker cells are known as a type of myocardial cells, and the pacemaker cells are cells in which most (90% to 100%) of the cells are composed of nodal cells. The myocardial cells used in the present disclosure are preferably cells consisting primarily of ventricular myocardial cells and are cells different from the pacemaker cells.

The proportion of the nodal cells in the myocardial cells used in the present disclosure is preferably 80% or less, more preferably 50% or less, and still more preferably 30% or less, and may be 20% or less.

The proportion of cells expressing a ventricular marker in the myocardial cells used in the present disclosure (that is, ventricular myocardial cells) is preferably 30% or more, more preferably 40% or more, and still more preferably 50% or more.

The ventricular myocardial cells, atrial myocardial cells, and nodal myocardial cells can be discriminated, for example, by the shape of an action potential, and a method described in Ma J. et al. Am J Physiol Heart Circ Physiol 301: H2006-H2017 2011 is exemplified. In addition, the cellular discrimination can also be carried out by immunostaining using the expression of a specific protein as a marker. In this case, examples of the marker for ventricular myocardial cells include MLC-2V, examples of the marker for atrial myocardial cells include MLC-2a, and examples of the marker for nodal myocardial cells include HCN.

In the present disclosure, the myocardial cells may be pre-cultured before the myocardial cells are treated with a culture medium containing a physiologically active substance of the sympathetic nervous system and/or a physiologically active substance of the parasympathetic nervous system. For example, in a case of using myocardial cells in a frozen state, the myocardial cells in a frozen state can be thawed, suspended in a thawing culture medium (for example, a thawing culture medium for iCell myocardial cells manufactured by FUJIFILM Cellular Dynamics, Inc. in a case where iCell myocardial cells 2.0 manufactured by FUJIFILM Cellular Dynamics, Inc. are used as the myocardial cells), and then seeded on a plate at 10,000 to 100,000 cells per well. The myocardial cells can be allowed to stand in a CO₂ incubator and then cultured in the CO₂ incubator for, for example, 1 day to 4 weeks, preferably 2 days to 2 weeks, with the addition of a maintenance culture medium (for example, an iCell myocardial cell maintenance culture medium manufactured by FUJIFILM Cellular Dynamics, Inc. in a case where iCell myocardial cells 2.0 manufactured by FUJIFILM Cellular Dynamics, Inc. are used as the myocardial cells). It should be noted that the culture medium may be exchanged with a serum-free culture medium on the day before the day of treating the myocardial cells with the culture medium containing a physiologically active substance of the sympathetic nervous system and/or a physiologically active substance of the parasympathetic nervous system.

In the present disclosure, the myocardial cells are treated with a culture medium containing a physiologically active substance of the sympathetic nervous system and/or a physiologically active substance of the parasympathetic nervous system before the myocardial cells are brought into contact with a drug. The culture medium preferably contains both a physiologically active substance of the sympathetic nervous system and a physiologically active substance of the parasympathetic nervous system.

The physiologically active substance of the sympathetic nervous system includes adrenergic receptor agonists, such as adrenaline, noradrenaline, isoproterenol, and terbutaline.

The physiologically active substance of the parasympathetic nervous system includes muscarinic receptor agonists, such as acetylcholine, muscarine, carbachol, bethanechol, cevimeline, and pilocarpine.

In a case where an adrenergic receptor agonist is used, the concentration of the adrenergic receptor agonist in the culture medium is preferably 0.05 µmol/L to 50 µmol/L, more preferably 0.1 µmol/L to 20 µmol/L, still more preferably 0.5 µmol/L to 10 µmol/L, particularly preferably 0.5 µmol/L to 5 µmol/L, and most preferably 0.5 µmol/L to 2 µmol/L.

In a case where a muscarinic receptor agonist is used, the concentration of the muscarinic receptor agonist in the culture medium is preferably 0.1 µmol/L to 100 µmol/L, more preferably 0.5 µmol/L to 50 µmol/L, still more preferably 1 µmol/L to 20 µmol/L, particularly preferably 1 µmol/L to 10 µmol/L, and most preferably 1 µmol/L to 5 µmol/L.

In a case where both a physiologically active substance of the sympathetic nervous system and a physiologically active substance of the parasympathetic nervous system are used in combination, the ratio of the concentrations of the physiologically active substance of the sympathetic nervous system and the physiologically active substance of the parasympathetic nervous system in the culture medium is preferably 0.1:1 to 1:20, more preferably 0.5:1 to 1:10, and still more preferably 1:1 to 1:5, and is, for example, 1:3.

The culture time for treating myocardial cells with a culture medium containing a physiologically active substance of the sympathetic nervous system and/or a physiologically active substance of the parasympathetic nervous system is generally 1 minute to 4 hours, preferably 5 minutes to 2 hours, more preferably 10 minutes to 1 hour, and still more preferably 10 minutes to 30 minutes.

The culture medium to which a physiologically active substance of the sympathetic nervous system and/or a physiologically active substance of the parasympathetic nervous system is added may be any medium that is generally used for culturing myocardial cells, and is not particularly limited, but in a case where the culture medium is serum-free or contains serum, the content of the serum is preferably 3% by volume or less and more preferably 1% by volume or less. A serum-free culture medium is particularly preferable as the culture medium, and a culture medium that does not contain albumin may also be used. Examples of the culture medium to which a physiologically active substance of the sympathetic nervous system and/or a physiologically active substance of the parasympathetic nervous system is added include, but are not particularly limited to, a myocardial cell serum-free assay culture medium, manufactured by FUJIFILM Cellular Dynamics, Inc.

The container for culturing myocardial cells may be a container generally used for cell culture and is not particularly limited thereto. Examples of the shape of the container include a petri dish, a plate, a bottle, a chamber, and a multi-well plate (for example, a 6-, 12-, 24-, 48-, 96-, or 384-well plate). Among the above, a multi-well plate is preferable, and from the viewpoint of throughput, a 48-well plate or a 96-well plate is particularly preferable.

The culture format of the myocardial cells is preferably plane culture.

In a case where a 48-well plate is used, the number of seeded myocardial cells is preferably 10,000 to 100,000 cells, more preferably 20,000 to 80,000 cells, and particularly preferably 30,000 to 60,000 cells per well.

In the present disclosure, myocardial cells are treated with a culture medium containing a physiologically active substance of the sympathetic nervous system and/or a physiologically active substance of the parasympathetic nervous system, and then the myocardial cells treated as described above are brought into contact with a drug. For example, a drug (including a drug candidate substance) can be added to a culture medium containing myocardial cells to thereby bring the myocardial cells into contact with the drug.

The drug (including a drug candidate substance) is not particularly limited and can be appropriately selected depending on the purpose. Examples of the drug include a compound such as a low-molecular-weight organic compound, a peptide, a protein, an antibody, a nucleic acid, a carbohydrate, a lipid, a cell extract, a cell culture supernatant, a plant extract, and a microbial product. A library of each of the compound, the peptide, the protein, and the antibody can also be used. For example, a compound library produced using a combinatorial chemistry technique can be used. In this regard, since the drug evaluation method according to the present disclosure allows for the evaluation of side effects, it is preferable to use, as a drug (candidate substance), a substance that has been confirmed to have a desired activity as the drug.

The drug (including a drug candidate substance) includes candidate substances for active ingredients of pharmaceutical products in all areas. For example, as the drug (including a drug candidate substance), it is possible to evaluate an agent for treating a heart disease. By heart disease is meant arrhythmia or any disease of the circulatory system that is accompanied by arrhythmia. The arrhythmia includes fast arrhythmia and slow arrhythmia.

Examples of the drug include, but are not particularly limited to, muscarinic receptor inhibitors, among which atropine, tiotropium, glycopyrronium, AF-DX116, and the like may be used.

Examples of the drug include, but are not particularly limited to, adrenergic receptor inhibitors, among which propranolol, atenolol, and the like may be used.

In the present disclosure, a drug is brought into contact with myocardial cells, and then the effect of the drug on the heart rate of the myocardial cells is evaluated.

As the evaluation of the effect of the drug on the heart rate of the myocardial cells, the inhibitory effect of the drug on the adrenergic receptor may be evaluated, or the inhibitory effect of the drug on the muscarinic receptor may be evaluated.

In the evaluation of the effect of the drug on the heart rate of the myocardial cells, for example, the beating rate (heart rate) can be calculated from the measurement results of the extracellular potential of the myocardial cells by taking an interval between sharp peaks indicating depolarization of the myocardial cells as the beating time (sec) and using a formula of heart rate (/min) = 60 /beating time (sec).

The extracellular potential can be measured, for example, by bringing myocardial cells into contact with a multiple electrode array. The multiple electrode array is an electrode in which a large number of microelectrodes, for example, 1 to 768 microelectrodes, are disposed on a substrate, and 1 to 384 wells, for example, 48 or 96 wells for cell culture are further disposed. For example, an MEA system (Maestro Pro, manufactured by Axion BioSystems, Inc.) is commercially available as a multiple electrode array (MEA) system which is capable of measuring the extracellular potential of the cultured cells. The multiple electrode array system is a system in which electrical signals from a plurality of cells can be simultaneously observed by a large number of microelectrodes disposed on a substrate, or cellular responses to stimuli can be observed by applying electrical stimuli to cells or tissues.

As another example, the beating of the myocardial cells may be detected by a standard electrophysiological method (for example, patch clamp), an elastic modulus detection device (for example, MicroTester (manufactured by CellScale Biomaterials Testing)), an imaging device, or a combination of these methods and devices.

In a case of using a multi-well plate having microelectrodes disposed in wells, the seeding density of myocardial cells in the vicinity of the electrode is preferably 100 to 10,000 cells/mm², more preferably 500 to 8,000 cells/mm², and particularly preferably 1,000 to 6,000 cells/mm². For the calculation of the seeding density of myocardial cells in the vicinity of the electrode, the myocardial cells can be seeded on a plate, cultured, fixed, and subjected to nuclear staining, and then the images obtained by imaging the electrode and the vicinity thereof under a microscope can be used. For example, paraformaldehyde can be used for the fixation of cells. For the nuclear staining, for example, Hoechst 33342 can be used, and the imaging can be carried out under a fluorescence microscope. In the images obtained by imaging the electrode and the vicinity thereof under a fluorescence microscope, the seeding density of the myocardial cells in the vicinity of the electrode can be calculated using analysis software.

According to the present disclosure, there is provided a reagent for carrying out the drug evaluation method according to the present disclosure, which contains an adrenergic receptor agonist and/or a muscarinic receptor agonist. The details of the adrenergic receptor agonist and/or the muscarinic receptor agonist are as described in the present specification.

According to the present disclosure, there is further provided a kit including the above-mentioned reagent according to the present disclosure and a culture medium. The kit according to the present disclosure may further include myocardial cells. The details of the culture medium and the myocardial cells are as described in the present specification.

The present disclosure will be described in more detail with reference to the following examples, but the present disclosure is not limited to these examples.

### Examples

Reference Example 1: Method and results of evaluating effect on heart rate in a case where muscarinic receptor agonist (carbachol) is added to serum-free culture medium

Human iPS cell-derived myocardial cells (iCell myocardial cells 2.0, manufactured by FUJIFILM Cellular Dynamics, Inc.) in a frozen state were thawed, suspended in a thawing culture medium (iCell myocardial cell thawing culture medium, manufactured by FUJIFILM Cellular Dynamics, Inc.), and then seeded at a cell density of 50,000 cells per well on a MEA plate (CytoView MEA 48, manufactured by Axion BioSystems, Inc.). The seeding density of myocardial cells in the vicinity of the electrode was 2,736 cells/mm². After the plate was allowed to stand in a CO₂ incubator for several hours, a maintenance culture medium (iCell myocardial cell maintenance culture medium, manufactured by FUJIFILM Cellular Dynamics, Inc.) containing serum was added, and the myocardial cells were cultured in the CO₂ incubator for about one week. The culture medium was exchanged with a serum-free culture medium (a myocardial cell serum-free assay culture medium, manufactured by FUJIFILM Cellular Dynamics, Inc.) on the day before the measurement.

On the day of measurement, the MEA plate on which the human iPS cell-derived myocardial cells were cultured was placed in an MEA system (Maestro Pro, manufactured by Axion BioSystems, Inc.), and the extracellular potential was measured after 20 minutes (medium control, carbachol concentration: 0 µmol/L). Subsequently, 0.01 µmol/L of carbachol (manufactured by Tokyo Chemical Industry Co., Ltd.) was added to the MEA plate, and then the extracellular potential was measured after 20 minutes from the addition. Similarly, carbachol was added to give concentrations of 0.1 µmol/L, 1 µmol/L, and 10 µmol/L, and then the extracellular potential was measured at each concentration after 20 minutes from the addition. The solvent for the carbachol to be added was a serum-free culture medium. The cells after the measurement were fixed with 4% paraformaldehyde and subjected to nuclear staining with Hoechst 33342, and images of the electrode and the vicinity thereof were taken under a fluorescence microscope (inverted microscope ECLIPSE Ti-U, manufactured by Nikon Corporation). The myocardial cells were detected from the acquired images using analysis software (ImageJ, NIH), and the seeding density of the myocardial cells in the vicinity of the electrode was calculated by normalizing the area of the imaged region. In addition, the seeding density of the myocardial cells in the vicinity of the electrode was calculated by the same method in the following experiments.

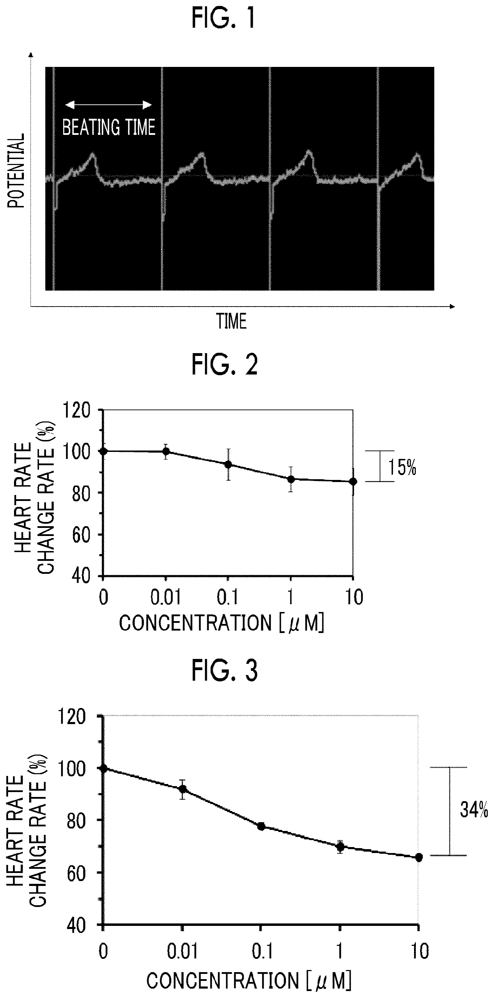

From the measurement results of the extracellular potential, the interval between sharp peaks indicating depolarization of human iPS cell-derived myocardial cells (Fig. 1) was taken as the beating time (sec), and the heart rate (/min) = 60/beating time (sec) was calculated. The addition of carbachol was confirmed to result in a decrease in heart rate of up to 15% (Fig. 2).

### Example 1: Method and results of evaluating decrease in heart rate by muscarinic receptor agonist (carbachol) using serum-free culture medium to which adrenergic receptor agonist (isoproterenol) is added

Human iPS cell-derived myocardial cells (iCell myocardial cells 2.0, manufactured by FUJIFILM Cellular Dynamics, Inc.) in a frozen state were thawed, suspended in a thawing culture medium (iCell myocardial cell thawing culture medium, manufactured by FUJIFILM Cellular Dynamics, Inc.), and then seeded at a cell density of 50,000 cells per well on a MEA plate (CytoView MEA 48, manufactured by Axion BioSystems, Inc.). The seeding density of myocardial cells in the vicinity of the electrode was 2,671 cells/mm². After the plate was allowed to stand in a CO₂ incubator for several hours, a maintenance culture medium (iCell myocardial cell maintenance culture medium, manufactured by FUJIFILM Cellular Dynamics, Inc.) containing serum was added, and the myocardial cells were cultured in the CO₂ incubator for about one week. The culture medium was exchanged with a serum-free culture medium (a myocardial cell serum-free assay culture medium, manufactured by FUJIFILM Cellular Dynamics, Inc.) on the day before the measurement.

On the day of measurement, the MEA plate on which human iPS cell-derived myocardial cells were cultured was placed in an MEA system (Maestro Pro, manufactured by Axion BioSystems, Inc.), 1 µmol/L of isoproterenol (manufactured by Tokyo Chemical Industry Co., Ltd.) was added to the culture medium, and then the extracellular potential was measured after 20 minutes from the addition (medium control, carbachol concentration: 0 µmol/L). Subsequently, 0.01 µmol/L of carbachol (manufactured by Tokyo Chemical Industry Co., Ltd.) was added to the MEA plate, and then the extracellular potential was measured after 20 minutes from the addition. Similarly, carbachol was added to give concentrations of 0.1 µmol/L, 1 µmol/L, and 10 µmol/L, and then the extracellular potential was measured at each concentration. The solvent for the carbachol to be added was a serum-free culture medium to which 1 µmol/L of isoproterenol was added, so that the concentration of isoproterenol in the serum-free culture medium did not change at the time of adding carbachol.

From the measurement results of the extracellular potential, the interval between sharp peaks indicating depolarization of human iPS cell-derived myocardial cells was taken as the beating time (sec), and the heart rate (/min) = 60/beating time (sec) was calculated. The addition of carbachol was confirmed to result in a decrease in heart rate of up to 34% (Fig. 3).

### Example 2: Method and results of evaluating decrease in heart rate by muscarinic receptor agonist (cevimeline) using serum-free culture medium to which adrenergic receptor agonist (isoproterenol) is added

In the same manner as in Example 1, evaluation was carried out using an MEA plate on which human iPS cell-derived myocardial cells were cultured. Specifically, the same operations as in Example 1 were carried out except that carbachol added in Example 1 was changed to cevimeline (manufactured by Sigma-Aldrich Co. LLC) and the concentration of cevimeline added was 0.1 µmol/L, 1 µmol/L, 10 µmol/L, and 100 µmol/L.

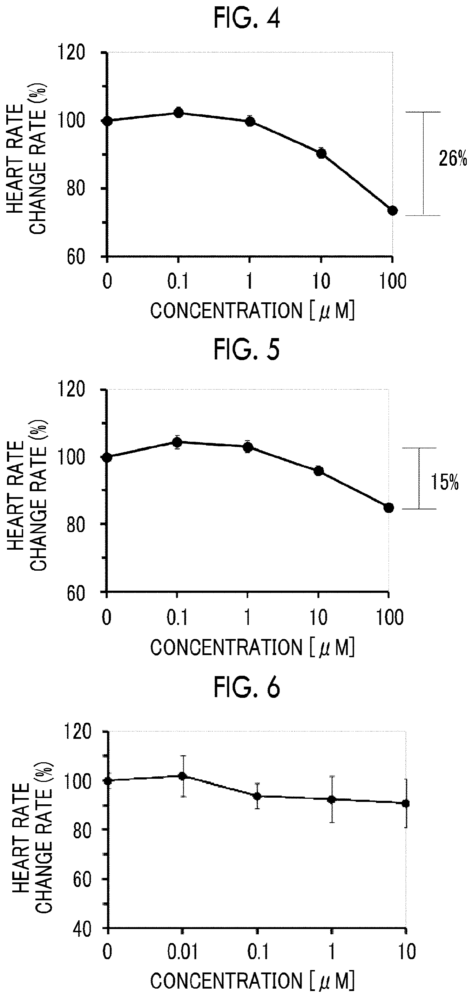

From the measurement results of the extracellular potential, the interval between sharp peaks indicating depolarization of human iPS cell-derived myocardial cells was taken as the beating time (sec), and the heart rate (/min) = 60/beating time (sec) was calculated. The addition of cevimeline was confirmed to result in a decrease in heart rate of up to 26% (Fig. 4).

Example 3: Method and results of evaluating decrease in heart rate by muscarinic receptor agonist (pilocarpine) using serum-free culture medium to which adrenergic receptor agonist (isoproterenol) is added
In the same manner as in Example 1, evaluation was carried out using an MEA plate on which human iPS cell-derived myocardial cells were cultured. Specifically, the same operations as in Example 1 were carried out except that carbachol added in Example 1 was changed to pilocarpine (manufactured by FUJIFILM Wako Pure Chemical Corporation) and the concentration of pilocarpine added was 0.1 µmol/L, 1 µmol/L, 10 µmol/L, and 100 µmol/L.

From the measurement results of the extracellular potential, the interval between sharp peaks indicating depolarization of human iPS cell-derived myocardial cells was taken as the beating time (sec), and the heart rate (/min) = 60/beating time (sec) was calculated. The addition of pilocarpine was confirmed to result in a decrease in heart rate of up to 15% (Fig. 5).

Comparative Example 1: Method and results of evaluating decrease in heart rate by muscarinic receptor agonist (carbachol) using maintenance culture medium
Human iPS cell-derived myocardial cells (iCell myocardial cells 2.0, manufactured by FUJIFILM Cellular Dynamics, Inc.) in a frozen state were thawed, suspended in a thawing culture medium (iCell myocardial cell thawing culture medium, manufactured by FUJIFILM Cellular Dynamics, Inc.), and then seeded at a cell density of 50,000 cells per well on a MEA plate (CytoView MEA 48, manufactured by Axion BioSystems, Inc.). The seeding density of myocardial cells in the vicinity of the electrode was 2,798 cells/mm². After the plate was allowed to stand in a CO₂ incubator for several hours, a maintenance culture medium (iCell myocardial cell maintenance culture medium, manufactured by FUJIFILM Cellular Dynamics, Inc.) containing serum was added, and the myocardial cells were cultured in the CO₂ incubator for about one week. The cells were cultured in the maintenance culture medium until the day of the measurement.

On the day of measurement, the MEA plate on which the human iPS cell-derived myocardial cells were cultured was placed in an MEA system (Maestro Pro, manufactured by Axion BioSystems, Inc.), and the extracellular potential was measured after 20 minutes (medium control, carbachol concentration: 0 µmol/L). Subsequently, 0.01 µmol/L of carbachol (manufactured by Tokyo Chemical Industry Co., Ltd.) was added to the MEA plate, and then the extracellular potential was measured after 20 minutes from the addition. Similarly, carbachol was added to give concentrations of 0.1 µmol/L, 1 µmol/L, and 10 µmol/L, and then the extracellular potential was measured at each concentration.

From the measurement results of the extracellular potential, the interval between sharp peaks indicating depolarization of human iPS cell-derived myocardial cells was taken as the beating time (sec), and the heart rate (/min) = 60/beating time (sec) was calculated. No clear decrease in heart rate due to the addition of carbachol was confirmed (Fig. 6).

Example 4: Method and results of evaluating increase in heart rate by muscarinic receptor inhibitor (atropine) using serum-free culture medium to which adrenergic receptor agonist (isoproterenol) and muscarinic receptor agonist (carbachol) are added
Human iPS cell-derived myocardial cells (iCell myocardial cells 2.0, manufactured by FUJIFILM Cellular Dynamics, Inc.) in a frozen state were thawed, suspended in a thawing culture medium (iCell myocardial cell thawing culture medium, manufactured by FUJIFILM Cellular Dynamics, Inc.), and then seeded at a cell density of 50,000 cells per well on a MEA plate (CytoView MEA 48, manufactured by Axion BioSystems, Inc.). The seeding density of myocardial cells in the vicinity of the electrode was 2,725 cells/mm². After the plate was allowed to stand in a CO₂ incubator for several hours, a maintenance culture medium (iCell myocardial cell maintenance culture medium, manufactured by FUJIFILM Cellular Dynamics, Inc.) containing serum was added, and the myocardial cells were cultured in the CO₂ incubator for about one week. The culture medium was exchanged with a serum-free culture medium (a myocardial cell serum-free assay culture medium, manufactured by FUJIFILM Cellular Dynamics, Inc.) on the day before the measurement.

On the day of measurement, the MEA plate on which human iPS cell-derived myocardial cells were cultured was placed in an MEA system (Maestro Pro, manufactured by Axion BioSystems, Inc.), 1 µmol/L of isoproterenol (manufactured by Tokyo Chemical Industry Co., Ltd.) and 3 µmol/L of carbachol (manufactured by Tokyo Chemical Industry Co., Ltd.) were added to the culture medium, and then the extracellular potential was measured after 20 minutes from the addition (medium control, atropine concentration: 0 µmol/L). Subsequently, 0.01 µmol/L of atropine (manufactured by Tokyo Chemical Industry Co., Ltd.) was added to the MEA plate, and then the extracellular potential was measured after 20 minutes from the addition. Similarly, atropine was added to give concentrations of 0.1 µmol/L and 1 µmol/L, and then the extracellular potential was measured at each concentration. The solvent for atropine to be added was a serum-free culture medium to which 1 µmol/L of isoproterenol and 3 µmol/L of carbachol were added, so that the concentrations of isoproterenol and carbachol in the serum-free culture medium did not change at the time of adding atropine.

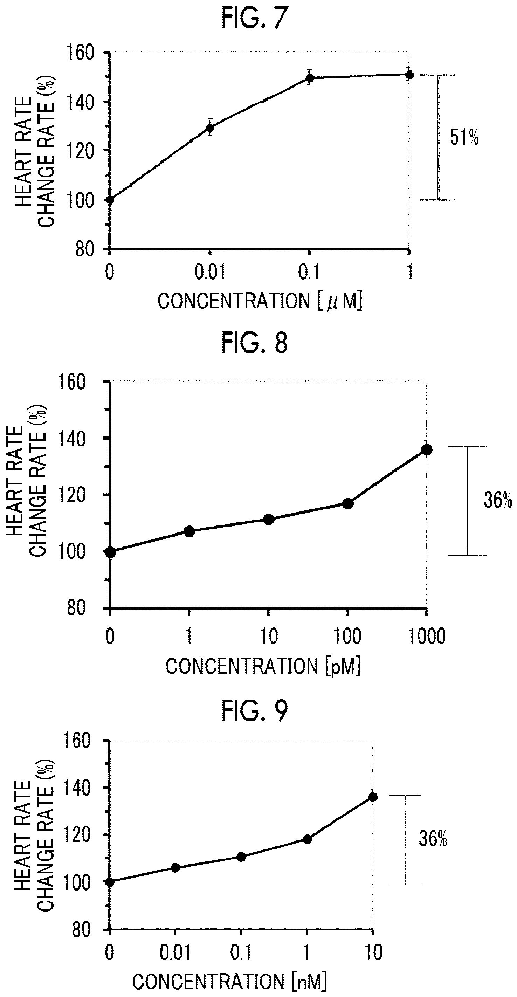

From the measurement results of the extracellular potential, the interval between sharp peaks indicating depolarization of human iPS cell-derived myocardial cells was taken as the beating time (sec), and the heart rate (/min) = 60/beating time (sec) was calculated. The addition of atropine was confirmed to result in an increase in heart rate of up to 51% (Fig. 7).

Example 5: Method and results of evaluating increase in heart rate by muscarinic receptor inhibitor (tiotropium) using serum-free culture medium to which adrenergic receptor agonist (isoproterenol) and muscarinic receptor agonist (carbachol) are added
In the same manner as in Example 4, evaluation was carried out using an MEA plate on which human iPS cell-derived myocardial cells were cultured. Specifically, the same operations as in Example 4 were carried out except that atropine added in Example 4 was changed to tiotropium (manufactured by Tokyo Chemical Industry Co., Ltd.) and the concentration of tiotropium added was 1 pmol/L, 10 pmol/L, 100 pmol/L, and 1,000 pmol/L.

From the measurement results of the extracellular potential, the interval between sharp peaks indicating depolarization of human iPS cell-derived myocardial cells was taken as the beating time (sec), and the heart rate (/min) = 60/beating time (sec) was calculated. The addition of tiotropium was confirmed to result in an increase in heart rate in a concentration-dependent manner, with an increase in heart rate of up to 36% being confirmed (Fig. 8).

Example 6: Method and results of evaluating increase in heart rate by muscarinic receptor inhibitor (glycopyrronium) using serum-free culture medium to which adrenergic receptor agonist (isoproterenol) and muscarinic receptor agonist (carbachol) are added
In the same manner as in Example 4, evaluation was carried out using an MEA plate on which human iPS cell-derived myocardial cells were cultured. Specifically, the same operations as in Example 4 were carried out except that atropine added in Example 4 was changed to glycopyrronium (manufactured by Tokyo Chemical Industry Co., Ltd.) and the concentration of glycopyrronium added was 0.01 nmol/L, 0.1 nmol/L, 1 nmol/L, and 10 nmol/L.

From the measurement results of the extracellular potential, the interval between sharp peaks indicating depolarization of human iPS cell-derived myocardial cells was taken as the beating time (sec), and the heart rate (/min) = 60/beating time (sec) was calculated. The addition of glycopyrronium was confirmed to result in an increase in heart rate in a concentration-dependent manner, with an increase in heart rate of up to 36% being confirmed (Fig. 9).

Example 7: Method and results of evaluating effect on heart rate in case where adrenergic receptor inhibitor (propranolol) is added using serum-free culture medium to which adrenergic receptor agonist (isoproterenol) and muscarinic receptor agonist (carbachol) are added
In the same manner as in Example 4, evaluation was carried out using an MEA plate on which human iPS cell-derived myocardial cells were cultured. Specifically, the same operations as in Example 4 were carried out except that the atropine added in Example 4 was changed to propranolol (manufactured by FUJIFILM Wako Pure Chemical Corporation) and the concentration of propranolol added was 0.1 nmol/L, 1 nmol/L, 10 nmol/L, and 100 nmol/L.

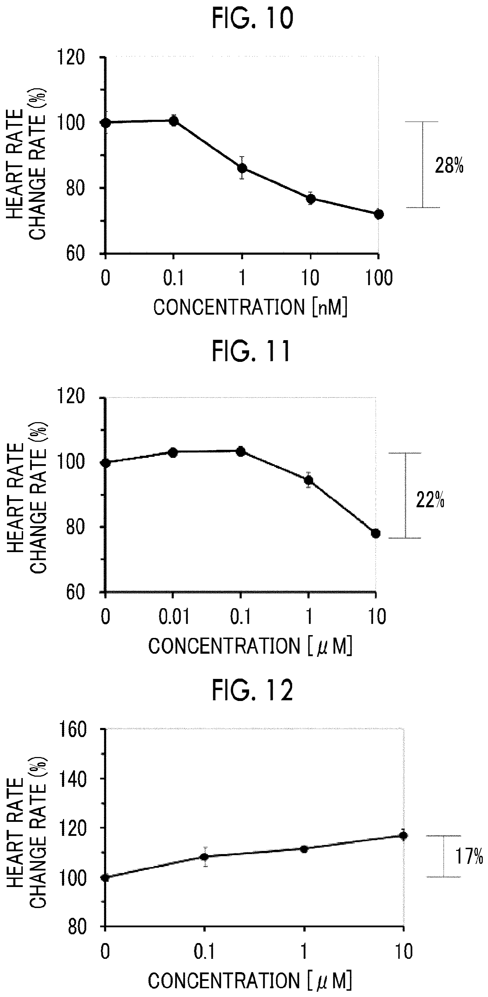

From the measurement results of the extracellular potential, the interval between sharp peaks indicating depolarization of human iPS cell-derived myocardial cells was taken as the beating time (sec), and the heart rate (/min) = 60/beating time (sec) was calculated. The addition of propranolol was confirmed to result in a decrease in heart rate in a concentration-dependent manner, with a decrease in heart rate of up to 28% being confirmed (Fig. 10).

Example 8: Method and results of evaluating effect on heart rate in case where adrenergic receptor inhibitor (atenolol) is added using serum-free culture medium to which adrenergic receptor agonist (isoproterenol) and muscarinic receptor agonist (carbachol) are added
In the same manner as in Example 4, evaluation was carried out using an MEA plate on which human iPS cell-derived myocardial cells were cultured. Specifically, the same operations as in Example 4 were carried out except that atropine added in Example 4 was changed to atenolol (manufactured by Tokyo Chemical Industry Co., Ltd.) and the concentration of atenolol added was 0.01 µmol/L, 0.1 µmol/L, 1 µmol/L, and 10 µmol/L.

From the measurement results of the extracellular potential, the interval between sharp peaks indicating depolarization of human iPS cell-derived myocardial cells was taken as the beating time (sec), and the heart rate (/min) = 60/beating time (sec) was calculated. The addition of atenolol was confirmed to result in a decrease in heart rate in a concentration-dependent manner, with a decrease in heart rate of up to 22% being confirmed (Fig. 11).

Example 9: Method and results of evaluating effect in case where drug (aspirin) that has no effect on heart rate is added using serum-free culture medium to which adrenergic receptor agonist (isoproterenol) and muscarinic receptor agonist (carbachol) are added.

Human iPS cell-derived myocardial cells (iCell myocardial cells 2.0, manufactured by FUJIFILM Cellular Dynamics, Inc.) in a frozen state were thawed, suspended in a thawing culture medium (iCell myocardial cell thawing culture medium, manufactured by FUJIFILM Cellular Dynamics, Inc.), and then seeded at a cell density of 50,000 cells per well on a MEA plate (CytoView MEA 48, manufactured by Axion BioSystems, Inc.). The seeding density of myocardial cells in the vicinity of the electrode was 2,783 cells/mm². After the plate was allowed to stand in a CO₂ incubator for several hours, a maintenance culture medium (iCell myocardial cell maintenance culture medium, manufactured by FUJIFILM Cellular Dynamics, Inc.) containing serum was added, and the myocardial cells were cultured in the CO₂ incubator for about one week. The culture medium was exchanged with a serum-free culture medium (a myocardial cell serum-free assay culture medium, manufactured by FUJIFILM Cellular Dynamics, Inc.) on the day before the measurement.

On the day of measurement, the MEA plate on which human iPS cell-derived myocardial cells were cultured was placed in an MEA system (Maestro Pro, manufactured by Axion BioSystems, Inc.), 1 µmol/L of isoproterenol (manufactured by Tokyo Chemical Industry Co., Ltd.) and 3 µmol/L of carbachol (manufactured by Tokyo Chemical Industry Co., Ltd.) were added to the culture medium, and then the extracellular potential was measured after 20 minutes from the addition (medium control, aspirin concentration: 0 µmol/L). Subsequently, 0.1 µmol/L of aspirin (manufactured by FUJIFILM Wako Pure Chemical Corporation) was added to the MEA plate, and then the extracellular potential was measured after 20 minutes from the addition. Similarly, aspirin was added to give concentrations of 1 µmol/L and 10 µmol/L, and then the extracellular potential was measured at each concentration. The solvent for aspirin to be added was a serum-free culture medium to which 1 µmol/L of isoproterenol and 3 µmol/L of carbachol were added, so that the concentrations of isoproterenol and carbachol in the serum-free culture medium did not change at the time of adding aspirin.

From the measurement results of the extracellular potential, the interval between sharp peaks indicating depolarization of human iPS cell-derived myocardial cells was taken as the beating time (sec), and the heart rate (/min) = 60/beating time (sec) was calculated. The increase in heart rate with the addition of aspirin was at most 17% (Fig. 12).

Comparative Example 3: Method and results of evaluating increase in heart rate by muscarinic receptor inhibitor (atropine) using maintenance culture medium.

Human iPS cell-derived myocardial cells (iCell myocardial cells 2.0, manufactured by FUJIFILM Cellular Dynamics, Inc.) in a frozen state were thawed, suspended in a thawing culture medium (iCell myocardial cell thawing culture medium, manufactured by FUJIFILM Cellular Dynamics, Inc.), and then seeded at a cell density of 50,000 cells per well on a MEA plate (CytoView MEA 48, manufactured by Axion BioSystems, Inc.). The seeding density of myocardial cells in the vicinity of the electrode was 2,662 cells/mm². After the plate was allowed to stand in a CO₂ incubator for several hours, a maintenance culture medium (iCell myocardial cell maintenance culture medium, manufactured by FUJIFILM Cellular Dynamics, Inc.) containing serum was added, and the myocardial cells were cultured in the CO₂ incubator for about one week. The cells were cultured in the maintenance culture medium until the day of the measurement.

On the day of measurement, the MEA plate on which the human iPS cell-derived myocardial cells were cultured was placed in an MEA system (Maestro Pro, manufactured by Axion BioSystems, Inc.), and the extracellular potential was measured after 20 minutes (medium control, atropine concentration: 0 µmol/L). Subsequently, 0.01 µmol/L of atropine (manufactured by Tokyo Chemical Industry Co., Ltd.) was added to the MEA plate, and then the extracellular potential was measured after 20 minutes from the addition. Similarly, atropine was added to give concentrations of 0.1 µmol/L and 1 µmol/L, and then the extracellular potential was measured at each concentration.

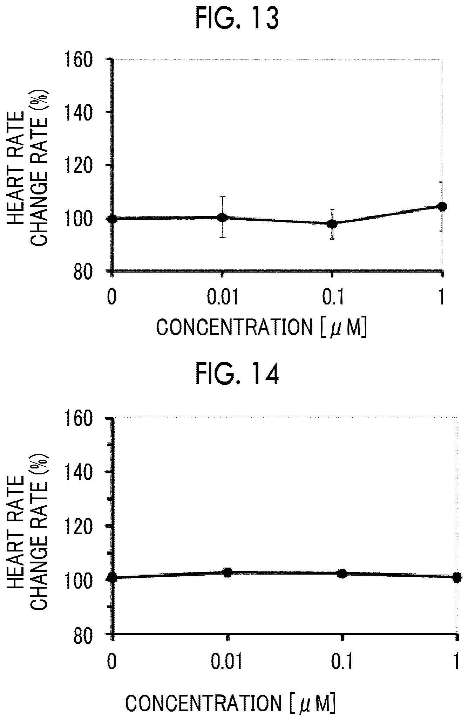

From the measurement results of the extracellular potential, the interval between sharp peaks indicating depolarization of human iPS cell-derived myocardial cells was taken as the beating time (sec), and the heart rate (/min) = 60/beating time (sec) was calculated. No clear increase in heart rate due to the addition of atropine was confirmed (Fig. 13).

Comparative Example 4: Method and results of evaluating increase in heart rate by muscarinic receptor inhibitor (atropine) using serum-free culture medium
Human iPS cell-derived myocardial cells (iCell myocardial cells 2.0, manufactured by FUJIFILM Cellular Dynamics, Inc.) in a frozen state were thawed, suspended in a thawing culture medium (iCell myocardial cell thawing culture medium, manufactured by FUJIFILM Cellular Dynamics, Inc.), and then seeded at a cell density of 50,000 cells per well on a MEA plate (CytoView MEA 48, manufactured by Axion BioSystems, Inc.). The seeding density of myocardial cells in the vicinity of the electrode was 2,703 cells/mm². After the plate was allowed to stand in a CO₂ incubator for several hours, a maintenance culture medium (iCell myocardial cell maintenance culture medium, manufactured by FUJIFILM Cellular Dynamics, Inc.) containing serum was added, and the myocardial cells were cultured in the CO₂ incubator for about one week. The culture medium was exchanged with a serum-free culture medium (a myocardial cell serum-free assay culture medium, manufactured by FUJIFILM Cellular Dynamics, Inc.) on the day before the measurement.

On the day of measurement, the MEA plate on which the human iPS cell-derived myocardial cells were cultured was placed in an MEA system (Maestro Pro, manufactured by Axion BioSystems, Inc.), and the extracellular potential was measured after 20 minutes (medium control, atropine concentration: 0 µmol/L). Subsequently, 0.01 µmol/L of atropine (manufactured by Tokyo Chemical Industry Co., Ltd.) was added to the MEA plate, and then the extracellular potential was measured after 20 minutes from the addition. Similarly, atropine was added to give concentrations of 0.1 µmol/L and 1 µmol/L, and then the extracellular potential was measured at each concentration after 20 minutes from the addition.

From the measurement results of the extracellular potential, the interval between sharp peaks indicating depolarization of human iPS cell-derived myocardial cells was taken as the beating time (sec), and the heart rate (/min) = 60 /beating time (sec) was calculated. No clear increase in heart rate due to the addition of atropine was confirmed (Fig. 14).

## Claims

1. A drug evaluation method, comprising:
treating myocardial cells with a culture medium containing a physiologically active substance of a sympathetic nervous system and/or a physiologically active substance of a parasympathetic nervous system;
bringing a drug into contact with the treated myocardial cells; and
evaluating an effect of the drug on a heart rate of the myocardial cells.

2. The method according to claim 1,
wherein the culture medium is serum-free.

3. The method according to claim 1 or 2,
wherein the physiologically active substance of the sympathetic nervous system includes an adrenergic receptor agonist.

4. The method according to claim 1 or 2,
wherein the physiologically active substance of the parasympathetic nervous system includes a muscarinic receptor agonist.

5. The method according to claim 1 or 2, further comprising:
evaluating an inhibitory effect of the drug on an adrenergic receptor.

6. The method according to claim 1 or 2, further comprising:
evaluating an inhibitory effect of the drug on a muscarinic receptor.

7. The method according to claim 1 or 2,
wherein the myocardial cells are human iPS cell-derived myocardial cells.

8. The method according to claim 1 or 2,
wherein the physiologically active substance of the parasympathetic nervous system is carbachol, and the physiologically active substance of the sympathetic nervous system is isoproterenol.

9. A reagent for carrying out the method according to claim 1 or 2, the reagent comprising:
an adrenergic receptor agonist and/or a muscarinic receptor agonist.

10. A kit comprising:
the reagent according to claim 9; and
a culture medium.

11. The kit according to claim 10, further comprising:
myocardial cells.
